# EUROPEAN PATENT APPLICATION

(11) **EP 2 228 444 A1**
(43) Date of publication of application: **15.09.2010**
(21) Application number: 09154684.6
(22) Date of filing: 09.03.2009
(51) Int. Cl.: C12N 15/11

(54) **microRNA for diagnostic and therapeutic purposes in cardiovascular diseases**

(71) Applicant: Julius-Maximilians-Universität Würzburg, 97970 Würzburg (DE)
(72) Inventor: Engelhardt, Stefan, 80999, Munich (DE); Jentzsch, Claudia, 97082, Wuerzburg (DE)
(74) Representative: Elbel, Michaela

(57) **Abstract**

The present invention relates to the use of microRNA and an antisense oligonucleotide for the manufacture of a medicament for the treatment and/or prevention and for the diagnosis of various diseases. An implant is also encompassed by the invention. The invention is directed to the use of microRNA for the manufacture of an implant. The present invention further relates to the use of microRNA or an antisense oligonucleotide against the microRNA for inducing a modification of a morphological phenotype of a cell. Additionally, the invention concerns a method for diagnosing a cardiovascular disease and a method for screening a pharmaceutically active compound for the treatment and/or prevention of a cardiovascular disease or a predisposition thereof.

SEQUENCE LISTING

<110>  Julius-Maximilians-Universitaet Wuerzburg

<120>  microRNA for diagnostic and therapeutic purposes in cardiovascular
       diseases

<130>  U30039

<160>  28

<170>  PatentIn version 3.3

<210>  1
<211>  137
<212>  RNA
<213>  Homo sapiens

<400>  1
guccccuccc cuaggccaca gccgagguca caaucaacau ucauuguugu cgguggguug      60

ugaggacuga ggccagaccc accgggggau gaaugucacu guggcugggc cagacacggc     120

uuaaggggaa uggggac                                                    137


<210>  2
<211>  71
<212>  RNA
<213>  Homo sapiens

<400>  2
uguucuaagg ugcaucuagu gcagauagug aaguagauua gcaucuacug cccuaagugc      60

uccuucuggc a                                                           71


<210>  3
<211>  92
<212>  RNA
<213>  Homo sapiens

<400>  3
cggcuggaca gcgggcaacg gaaucccaaa agcagcuguu gucuccagag cauuccagcu      60

gcgcuuggau uucguccccu gcucuccugc cu                                    92


<210>  4
<211>  89
<212>  RNA
<213>  Homo sapiens

<400>  4
cuggccucca gggcuuugua caugguaggc uuucauucau ucguuugcac auucggugaa      60

ggucuacugu gugccaggcc cugugccag                                        89

<210> 5
<211> 91
<212> RNA
<213> Homo sapiens

<400> 5
auuuucauca ccuagggauc uuguuaaaaa gcagauucug auucagggac caagauucug 60

cauuuuuagc aaguucucaa gugaugcuaa u 91


<210> 6
<211> 97
<212> RNA
<213> Homo sapiens

<400> 6
ugcagggagg uauuaaguug gugcaaaagu aauugugauu uuugccauua aaaguaacga 60

caaaacuggc aauuacuuuu gcaccaaacc ugguauu 97


<210> 7
<211> 97
<212> RNA
<213> Homo sapiens

<400> 7
ugugaugugu auuagguuug ugcaaaagua auugggguuu uuugccguua aaaguaaugg 60

caaaacuggc aauuacuuuu gcaccaaacu aauauaa 97


<210> 8
<211> 97
<212> RNA
<213> Homo sapiens

<400> 8
ccuagaaugu uauuaggucg gugcaaaagu aauugcgagu uuuaccauua cuuucaaugg 60

caaaacuggc aauuacuuuu gcaccaacgu aauacuu 97


<210> 9
<211> 96
<212> RNA
<213> Homo sapiens

<400> 9
aaccauucaa auauaccaca guuuguuuaa ccuuuugccu guugguugaa gaugccuuuc 60

aacaggugac ugguuagaca aacuguggua uauaca 96


<210> 10
<211> 96

<212>  RNA
<213>  Homo sapiens

<400>  10
ggagugaacu cagaugugga gcacuaccuu ugugagcagu gugacccaag gccuguggac      60

aggguaagcu gaaccucuga uaaaacucug aucuau                                96


<210>  11
<211>  96
<212>  RNA
<213>  Homo sapiens

<400>  11
agauaaaucu auagacaaaa uacaaucccg gacaacaaga agcuccuaua gcuccuguag      60

cuucuugugc ucuaggauug uauuuuguuu auauau                                96


<210>  12
<211>  98
<212>  RNA
<213>  Homo sapiens

<400>  12
aagucacgug cuguggcucc agcuucauag gaaggcucuu gucugucagg caguggaguu      60

acuuacagac aagagccuug cucaggccag cccugccc                              98


<210>  13
<211>  100
<212>  RNA
<213>  Homo sapiens

<400>  13
gggccaaggu gggccagggg ugguguuggg acagcuccgu uuaaaaaggc aucuccaaga      60

gcuuccauca aaggcugccu cuuggugcag cacagguaga                           100


<210>  14
<211>  95
<212>  RNA
<213>  Homo sapiens

<400>  14
ugcucggcug uuccuagggu guuucucuca ucucuggucu auaauggguu aaauaguaga      60

gaugagggca acacccuagg aacagcagag gaacc                                 95


<210>  15
<211>  97
<212>  RNA
<213>  Homo sapiens

<400>  15
caucauaagg agccuagacu ucccauuuga agguggccau uuccuaccac cuucaaaugg      60

uaaguccagg cuccuucuga uucaauaaau gaggagc                               97


<210>  16
<211>  97
<212>  RNA
<213>  Homo sapiens

<400>  16
aaacccacac cacugcauuu uggccaucga ggguuggggc uuggugucau gccccaagau      60

aaccagcacc ccaacuuugg acagcaugga uuagucu                               97


<210>  17
<211>  97
<212>  RNA
<213>  Homo sapiens

<400>  17
cugcuccuuc ucccauaccc auugcauauc ggaguuguga auucucaaaa caccuccugu      60

gugcauggau uacaggaggg ugagccuugu caucgug                               97


<210>  18
<211>  109
<212>  RNA
<213>  Homo sapiens

<400>  18
gcuuuuauau uguagguuuu ugcucaugca ccaugguugu cugagcaugc agcaugcuug      60

ucugcucaua ccccaugguu ucugagcagg aaccuucauu gucuacugc                 109


<210>  19
<211>  71
<212>  RNA
<213>  Homo sapiens

<400>  19
uguguuaagg ugcaucuagu gcaguuagug aagcagcuua gaaucuacug cccuaaaugc      60

cccuucuggc a                                                           71


<210>  20
<211>  94
<212>  RNA
<213>  Homo sapiens

<400>  20
caugcugugu gugguacccu acugcagaca guggcaauca uguauaauua aaaaugauug      60

guacgucugu ggguagagua cugcaugaca caug                                    94

<210>  21
<211>  91
<212>  RNA
<213>  Homo sapiens

<400>  21
caugcugugu gugguacccu acugcagaca guggcaauca uguauaauua aaaaugauug      60

guacgucugu ggguagagua cugcaugaca c                                     91

<210>  22
<211>  75
<212>  RNA
<213>  Homo sapiens

<400>  22
gugguacccu acugcagacg uggcaaucau guauaauuaa aaaugauugg uacgucugug      60

gguagaguac ugcau                                                       75

<210>  23
<211>  96
<212>  RNA
<213>  Homo sapiens

<400>  23
ccucaguaag accaagcuca gugugccauu uccuugucug uagccauguc uaugggcucu      60

ugaguuggcc aucugaguga gggccugcuu auucua                                96

<210>  24
<211>  98
<212>  RNA
<213>  Homo sapiens

<400>  24
aagucacgug cuguggcucc agcuucauag gaaggcucuu gucugucagg caguggaguu      60

acuuacagac aagagccuug cucaggccag cccugccc                              98

<210>  25
<211>  102
<212>  RNA
<213>  Homo sapiens

<400>  25
gguccucuga cucucuucgg ugacggguau ucuugggugg auaauacgga uuacguuguu      60

auugcuuaag aauacgcgua gucgaggaga guaccagcgg ca                        102

<210>  26
<211>  83
<212>  RNA
<213>  Homo sapiens

<400>  26
   cugacuaugc cuccccgcau ccccuagggc auugguguaa agcuggagac ccacugcccc      60
   aggugcugcu ggggguugua guc                                              83


<210>  27
<211>  76
<212>  RNA
<213>  Homo sapiens

<400>  27
uacuugaaga gaaguuguuc gugguggauu cgcuuuacuu augacgaauc auucacggac      60

aacacuuuuu ucagua                                                     76


<210>  28
<211>  63
<212>  RNA
<213>  Homo sapiens

<400>  28
aagaaauggu uuaccguccc acauacauuu ugaauaugua ugugggaugg uaaaccgcuu      60

cuu                                                                   63

## Description

### Field of the invention

The present invention relates to the field of microRNA, in particular to microRNAs and antisense oligonucleotides against microRNAs for the diagnosis, prevention and/or therapy. Especially, the present invention relates to the use of microRNAs and its antisense oligonucleotides for the manufacture of a medicament for the treatment and/or prevention and for the diagnosis of various diseases.

### Background of the invention

microRNAs are a broad class of small non-coding single stranded RNAs that control diverse biological processes including major signaling pathways, like developmental timing, hematopoietic cell differentiation, apoptosis, cell proliferation, and organ development (Kim, 2005). microRNAs regulate the expression of complementary target mRNAs by post-transcriptional gene silencing, which leads to mRNA cleavage or translational repression. With more than 200 members per species in higher eukaryotes, miRNAs are one of the largest gene family accounting for about 1 % of the genome (Bartel, 2004 A). More than one third of all human genes are targeted by miRNAs (Lewis et al., 2005). miRNAs and their targets seem to form complex regulatory networks. For example, a single microRNA regulates many different mRNA targets, and several different microRNAs control a single mRNA target. Consequently, the unique combination of miRNAs that are expressed in each cell type might affect the utilization of thousands of mRNAs (Lewis et al., 2003; Bartel et al., 2004 B; Kim, 2005).

Dysregulation of microRNAs in various disease entities is caused by alterations in the genome (Mi et al., 2007). Differential expression or viral infections microRNA change function into tumor suppressors or oncogenes in some cases. microRNAs were recently implicated in the regulation of diverse cardiac functions in a series of elegant genetic studies (Care et al., 2007; Yang et al., 2007). Although these studies help to delineate the role of microRNA in heart physiology, growth and morphogenesis, detailed molecular mechanism for microRNAs in disease pathways *in vivo* are purely understood. Single stranded oligonucleotide antagonists against microRNAs have been shown to silence endogenous microRNAs *in vitro* and *in vivo* by affecting target mRNA. microRNA silencing results in modified protein levels and metabolism (Kruetzfeld et al., 2005).

Nevertheless, the therapeutic potential of specific microRNA and their antagonists in different disease models remains to be established.

The solution to this problem is achieved by providing the embodiments characterized by the claims, and described further below.

### Summary of the invention

A first aspect of the present invention relates to the use of a microRNA selected from the group consisting of SEQ ID NO: 10, 15, 20, 21, 22, 25, 27, 28, hsa-miR-299-5p, and hsa-miR-509 for the manufacture of a medicament for the treatment and/or prevention of a disease selected from the group consisting of lesions, injury, neuronal degeneration, paraplegia, cardiovascular disease, and cicatrization of organ, connective tissue or skin.

A second aspect of the present invention relates to the use of a microRNA selected from the group consisting of SEQ ID NO: 10, 15, 20, 21, 22, 25 to 28, hsa-miR-299-5p, hsa-miR-324-5p, and hsa-miR-509 for the manufacture of an implant for plastic surgery, organ and/or tissue replacement.

A third aspect of the present invention relates to an implant obtainable by a method comprising the steps of:
(a) providing at least one cell; and
(b) contacting a microRNA selected from the group consisting of SEQ ID NO: 10, 15, 20, 21, 22, 25 to 28, hsa-miR-299-5p, hsa-miR-324-5p, and hsa-miR-509 with the cell;
wherein a modification of a morphological phenotype of the cell is induced.

Another aspect of the present invention relates to the use of a microRNA or an antisense oligonucleotide against the microRNA for inducing a modification of a morphological phenotype of a cell, wherein the microRNA is selected from the group consisting of SEQ ID NO: 10, 15, 20, 21, 22, 25 to 28, hsa-miR-299-5p, hsa-miR-324-5p, and hsa-miR-509.

A further aspect of the present invention relates to the use of a microRNA selected from the group consisting of SEQ ID NO: 1, 4 to 24, hsa-miR-18a*, hsa-miR-191*, hsa-miR-493-5p, hsa-miR-548a, hsa-miR-767-5p, hsa-miR-509, an antisense oligonucleotide against SEQ ID NO: 1, 4 to 24, hsa-miR-493-5p, hsa-miR-548a, hsa-miR-767-5p, and an antisense oligonucleotide against hsa-miR-509 for the manufacture of a medicament for the treatment and/or prevention of a cardiovascular disease.

Another aspect of the present invention relates to the use of a microRNA selected from the group consisting of SEQ ID NO: 1, 4 to 24, hsa-miR-18a*, hsa-miR-191*, hsa-miR-493-5p, hsa-miR-548a, hsa-miR-767-5p, hsa-miR-509, an antisense oligonucleotide against SEQ ID NO: 1, 4 to 24, hsa-miR-493-5p, hsa-miR-548a, hsa-miR-767-5p, and an antisense oligonucleotide against hsa-miR-509 for the diagnosis of a cardiovascular disease or a predisposition thereof.

Still another aspect of the present invention relates to a method for diagnosing a cardiovascular disease, the method comprises the steps of:
(a) providing a sample of a patient supposed to suffer from the cardiovascular disease; and
(b) measuring a level of an endogenous microRNA of the sample, wherein a modified level of a microRNA selected from the group consisting of SEQ ID NO: 1, 4 to 18, hsa-miR-18a*, hsa-miR-191*, hsa-miR-493-5p, hsa-miR-548a, and hsa-miR-767-5p, in comparison to a control sample indicates a proliferative cardiovascular disease or a predisposition thereof, and/or a modified level of a microRNA selected from the group consisting of SEQ ID NO: 4, 11, 19 to 24, hsa-miR-493-5p, and hsa-miR-509, in comparison to a control sample indicates a cardiovascular disease that relates to apoptosis or proliferation inhibition or a predisposition thereof.

A further aspect of the present invention relates to a method for screening a pharmaceutically active compound for the treatment and/or prevention of a cardiovascular disease or a predisposition thereof, the method comprises the steps of:
(a) providing a sample comprising a microRNA selected from the group consisting of SEQ ID NO: 1, 4 to 24, hsa-miR-18a*, hsa-miR-191*, hsa-miR-493-5p, hsa-miR-548a, hsa-miR-767-5p, and hsa-miR-509;
(b) contacting a candidate substance with the sample; and
(c) determining the effect of the candidate substance on the sample;
wherein a modification of the microRNA indicates a pharmaceutically active compound.

The invention will be more apparent from the disclosure of the following description together with the figures and sequence listing.

### Description of the drawings

Figure 1 displays a computerized cell size analysis of α-actinin- and DAPI-stained neonatal rat cardiomyocytes (NRCM) culture under hypertrophy-inducing condition (left). NRCMs are recognized according to their α-actinin staining and thereby, other cells within the NRCM culture are excluded (right).
Figure 2 displays immunostained NRCMs cultured under basal condition and hypertrophy-inducing condition.
Figure 3 displays quantitative results of candidate microRNAs that induce cardiomyocyte cell growth. The cell size is normalized to the control. Light gray: basal condition; dark gray: hypertrophy-inducing condition by 50 µM PE.
Figure 4 displays quantitative results of candidate miRNAs that inhibit cardiomyocyte cell growth. The cell size is normalized to the control. Light gray: basal condition; dark gray: hypertrophy-inducing condition by 50 µM PE.
Figure 5 shows cell morphologies induced by specific microRNAs. microRNA-137 induces cytoskeletal destruction. miR-299-5p and miR-324-5p enhance elongation and branching of NRCMs. The cell size is normalized to the control, i.e. for miR-299-5p, basal: 1,00; 50 µM PE: 0,00.
Figure 6 shows cell morphologies induced by specific microRNAs. miR-382, miR-509, miR-617 induce enhanced elongation and branching of NRCMs. miR-555 induces reduction of NRCM branching.

### Detailed description of the invention

In a first aspect the present invention relates to the use of a microRNA selected from the group consisting of SEQ ID NO: 10, 15, 20, 21, 22, 25, 27, 28, hsa-miR-299-5p, and hsa-miR-509 for the manufacture of a medicament for the treatment and/or prevention of a disease selected from the group consisting of lesions, injury, neuronal degeneration, paraplegia, cardiovascular disease, and cicatrization of an organ, connective tissue or skin.

Enhancement of cellular elongation and branching by microRNA induces cellular proliferation, sprouting and site directed growth of cells. These cellular morphological modifications provide a basis for cellular repair mechanisms, regeneration and neoplasm. Therefore, in approaches for treating apoptotic or degenerative diseases microRNAs are used for the manufacture of medicaments according to the invention.

The manufacture of a medicament for the treatment and/or prevention of a disease selected from the group consisting of lesions, injury, neuronal degeneration, paraplegia, and cicatrization of an organ, connective tissue or skin using SEQ ID NO: 10, 15, 20, 21, 22, 25, 27, 28, hsa-miR-299-5p, and hsa-miR-509 is a new medical indication.

Cellular morphological modifications by microRNA are useful for the following therapeutic purposes:
Elongated shape: In dilated cardiomyopathy, wherein the ventricular diameter increases, an elongated shape of cardiomyocytes has been described (increase of long-axis diameter). In contrast, hypertrophic cardiomyopathy cardiomyocytes are mainly thicker, i.e. their short axis diameter increases. The underlying mechanisms for this shape change are largely unknown. Inhibiting cardiomyocyte elongation may represent a way to prevent or cure cardiac diseases that displays dilatation of the ventricles. Examples are idiopathic and inherited forms of dilated cardiomyopathy.

Cellular outgrowths/roundish morphology: Intercellular communication through gap junctions between cardiomyocytes is essential for electrical conduction as well as the formation of an ordered tissue structure. Cellular outgrowth may provide the basis for these intercellular communications and formation. In cardiac disease, both intercellular communication through gap junctions and the order of the tissue architecture are disturbed. Thus promoting cellular outgrowths or inhibiting the impairment of cellular outgrowth formation may represent a therapeutic measure in cardiac disease.

Cytoskeletal architecture: The ordered alignment of contractile proteins is mandatory for cardiomyocyte contractility and thus cardiac function. Means to preserve the cytoskeletal architecture may be employed to treat heart disease or prevent deterioration of existing disease.

In another aspect the present invention relates to the use of a microRNA selected from the group consisting of SEQ ID NO: 10, 15, 20, 21, 22, 25 to 28, hsa-miR-299-5p, hsa-miR-324-5p, and hsa-miR-509 for the manufacture of an implant for plastic surgery, organ and/or tissue replacement.

In another aspect the present invention relates to an implant obtained by a method comprising the steps of:
(a) providing at least one cell; and
(b) contacting a microRNA selected from the group consisting of SEQ ID NO: 10, 15, 20, 21, 22, 25 to 28, hsa-miR-299-5p, hsa-miR-324-5p, and hsa-miR-509 with the cell to induce a modification of a morphological phenotype of the cell.

The term "implant" as used herein is a biomaterial that replaces and acts as a biological structure. The term "implant" includes a single cell, an accumulation of cells, a tissue, an organ, and organ systems. In an implant the biomaterial can be combined with non-biomaterial, like a medical device or materials, such as titanium, silicone or apatite. The implant can also be combined with bioactive substances for e.g. drug delivery.

The microRNA selected from the group consisting of SEQ ID NO: 10, 15, 20, 21, 22, 25 to 28, hsa-miR-299-5p, hsa-miR-324-5p, and hsa-miR-509 induces a modification of the morphological phenotype of the cell. Based on this induced modification implants can be cultivated. Enhancement of cellular elongation and branching induced by microRNA are prerequisites for implant preparation and result in sprouting and site directed growth of cells.

In a preferred embodiment, the method comprises the further step of cultivating the modified cell in an environment, which induces the modification. In a preferred embodiment, the method comprises the further step of giving the cell a desired shape by the environment of the cell. Preferably, the environment is a mold. In a preferred embodiment, the method comprises the further step of harvesting the shaped cell.

In another aspect the present invention relates to the use of a microRNA or an antisense oligonucleotide against the microRNA for inducing a modification of a morphological phenotype of a cell, wherein the microRNA is selected from the group consisting of SEQ ID NO: 10, 15, 20, 21, 22, 25 to 28, hsa-miR-299-5p, hsa-miR-324-5p, and hsa-miR-509.

The term "morphological phenotype" as used herein refers to the appearance of a cell including the size, shape and distribution of membranes, organelles, nucleus, and cytoskeleton. The term "cytoskeletal reorganization" as used herein refers to any kind of assembly, decomposition, and reconstruction of the cytoskeleton and its constituent parts, like actin filaments and microtubuli. The term "cell" as used herein refers to a single cell and an accumulation of cells, like cell cultures, explants, tissue, organs, and non-human organisms.

Cardiomyocytes display an elongated cell shape and cellular outgrowths after treatment with SEQ ID NO: 15, 20 to 22, 27, hsa-mir-299-5p, hsa-mir-324-5p, and hsa-mir-509, compared to control-treated cardiomyocytes. Cellular outgrowths are protrusions of the cell body which are detected by immunofluorescent detection of α-actinin, also called branching.

Cardiomyocytes display a roundish morphology after treatment with SEQ ID NO: 10 compared to control-treated cardiomyocytes. Roundish cardiomyocytes display less cellular outgrowths as control-treated cells, and some are completely devoid of significant cellular outgrowths as determined by staining for a-actinin.

Cardiomyocytes display destruction of their cytoskeletal architecture after treatment with a SEQ ID NO: 25 compared to control-treated cardiomyocytes.

In a preferred embodiment the modification is selected from the group consisting of reshaping, elongation, branching, rounding, protuberance and cytoskeletal reorganization.

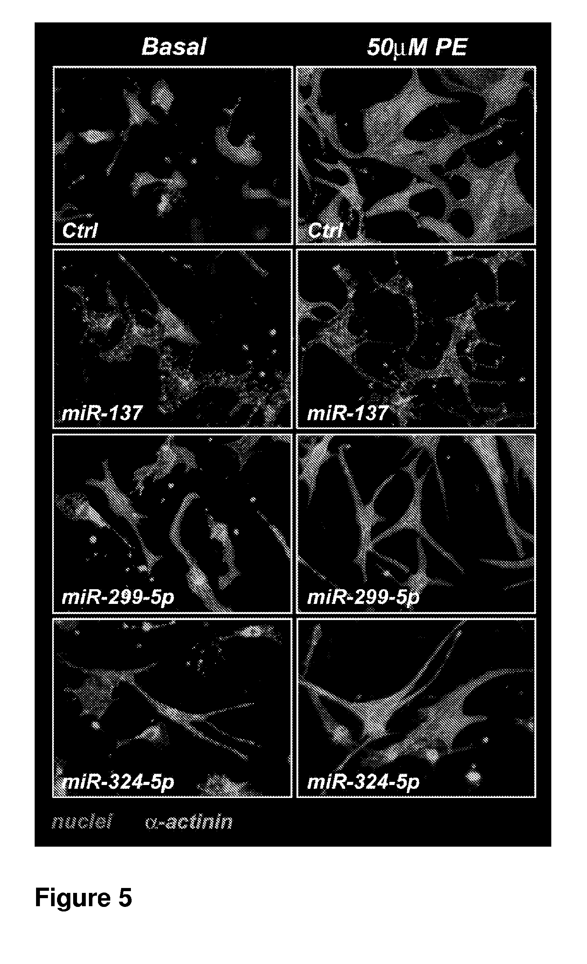

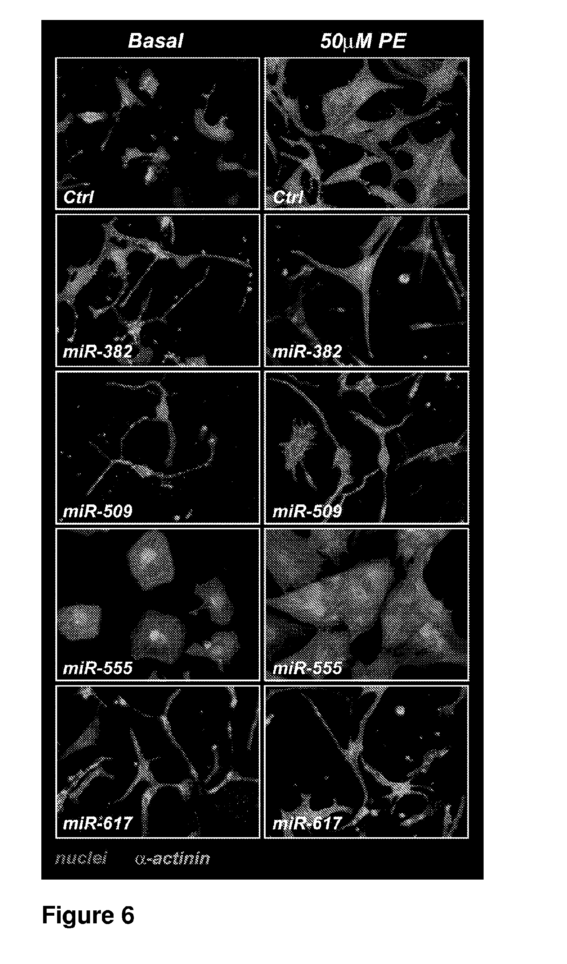

In another preferred embodiment the microRNA or the antisense against the microRNA is used to enhance or inhibit elongation and branching, and the microRNA is selected from the group consisting of SEQ ID NO: 10, 15, 20, 21, 22, 26, 27, 28, hsa-miR-299-5p, hsa-miR-324-5p, and hsa-miR-509 (Figures 5 and 6). SEQ ID NO: 10 reduces cellular elongation and branching. SEQ ID NO: 15, 20, 21, 22, 26, 27, 28, hsa-miR-299-5p, hsa-miR-324-5p, and hsa-miR-509 enhances cellular elongation and branching.

In a further preferred embodiment the SEQ ID NO: 25 or the antisense against SEQ ID NO: 25 is used to reorganize cytoskeleton (Figure 5). SEQ ID NO: 25 destructs the cytoskeleton.

In another aspect the present invention relates to the use of a microRNA selected from the group consisting SEQ ID NO: 1, 4 to 24, hsa-miR-18a*, hsa-miR-191*, hsa-miR-493-5p, hsa-miR-548a, hsa-miR-767-5p, hsa-miR-509, an antisense oligonucleotide against SEQ ID NO: 1, 4 to 24, hsa-miR-493-5p, hsa-miR-548a, hsa-miR-767-5p, and an antisense oligonucleotide against hsa-miR-509 for the manufacture of a medicament for the treatment and/or prevention of a cardiovascular disease.

In a further aspect the present invention relates to the use of a microRNA selected from the group consisting of SEQ ID NO: 1, 4 to 24, hsa-miR-18a*, hsa-miR-191*, hsa-miR-493-5p, hsa-miR-548a, hsa-miR-767-5p, hsa-miR-509, an antisense oligonucleotide against SEQ ID NO: 1, 4 to 24, hsa-miR-493-5p, hsa-miR-548a, hsa-miR-767-5p, and an antisense oligonucleotide against hsa-miR-509 for the diagnosis of a cardiovascular disease or a predisposition thereof.

The term "microRNA" as used herein refers to a single stranded RNA of about 19 to 25 nucleotides in length that is generated from endogenous hairpin-shaped transcripts by the RNase-III-type enzyme Dicer. microRNAs are present in one arm of the hairpin precursor, which lacks large internal loops or bulges.

The term "antisense oligonucleotide" as used herein refers to a complementary strand of nucleic acids that hybridizes with its sense strand. The term "antisense oligonucleotide" comprises enzyme dependent antisense oligonucleotides, steric blocking antisense oligonucleotide, ribonucleic and deoxyribonucleic sequences, artificial and endogenous antisense oligonucleotides, coding and non-coding sequences, double and single stranded sequences regardless of length and formation.

The term "cardiovascular disease" as used herein refers to a class of diseases, disorders and conditions that involves the heart or blood vessels. The term "cardiovascular disease" comprises disease types, like aneurysms, angina, arteriosclerosis, stroke, cerebrovascular diseases, congestive heart failure, coronary artery disease, myocardial infarction and peripheral vascular disease. Also encompassed are inflammatory, degenerative, metabolic, hereditary and accidental diseases.

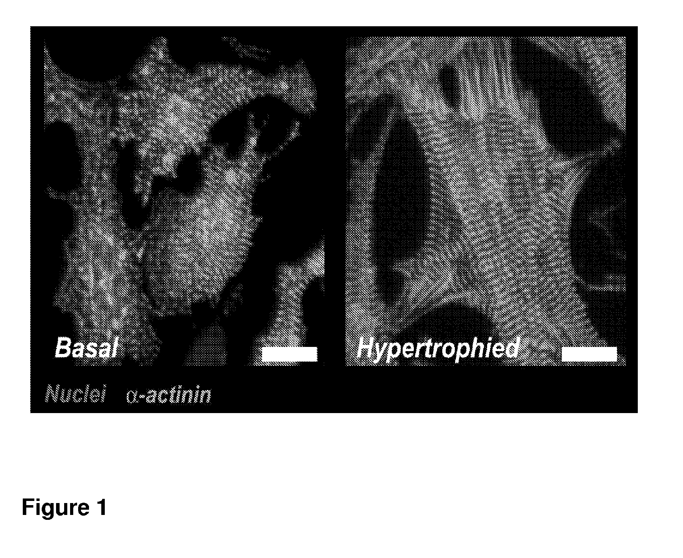

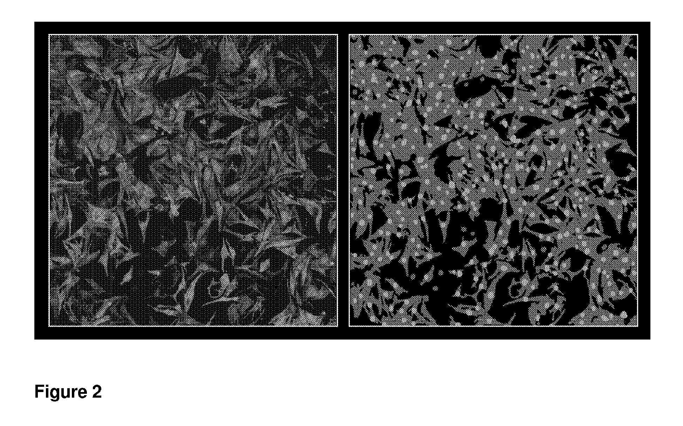

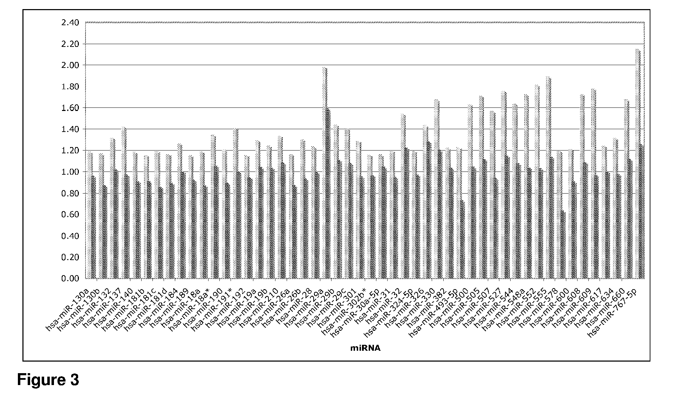

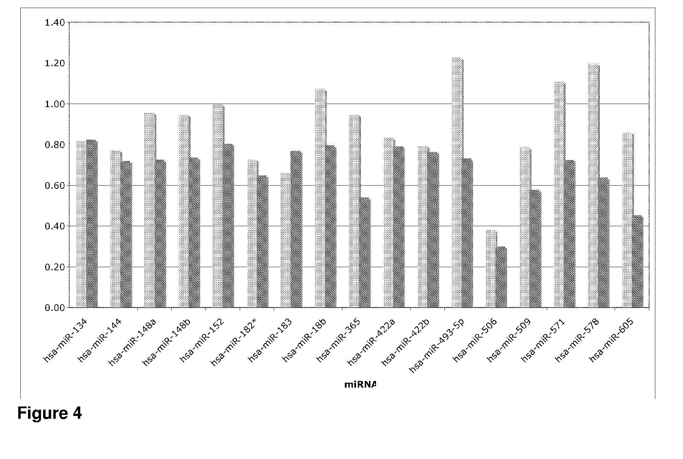

For the identification of functionally relevant microRNAs a high-throughput screening assay was developed by the inventors in a 96-well format. By means of this assay it was possible to screen a library of 471 human microRNAs directly on the functional level in primary cell culture. Computational-based microscopic analysis of cardiomyocyte cell size was conducted (Figure 1). An *in vitro* culture of transfected cardiomyocytes under basal and hypertrophy-inducing conditions (Figure 2) was used to classify the investigated miRNAs according to their function. The results show that pathological growth and hypertrophy of cardiomyocytes are key events during the development and progression of cardiovascular diseases, like heart failure (Figures 3 and 4). After the screening procedure approximately 15% of the investigated miRNAs displayed a phenotypic effect either by promoting or inhibiting cell growth. They evidence that microRNA selected from the group consisting of SEQ ID NO: 1, 5 to 24, hsa-miR-18a*, hsa-miR-191*, hsa-miR-493-5p, hsa-miR-548a, hsa-miR-767-5p, hsa-miR-509, an antisense oligonucleotide against SEQ ID NO: 1, 5 to 24, hsa-miR-493-5p, hsa-miR-548a, hsa-miR-767-5p, and an antisense oligonucleotide against hsa-miR-509 plays an important role in cardiovascular disease processes. These microRNAs modify cardiomyocyte cell proliferation by regulating gene expression via mRNA cleavage and translational repression. A defined set of miRNAs induced strong promotion (> 2 fold) or inhibition (< 0.5 fold) of cardiomyocyte cell growth. Under basal conditions SEQ ID NO: 1, 5 to 18, hsa-miR-493-5p, and hsa-miR-548a induce cardiomyocyte cell growth, which indicates a prohypertrophic phenotype and a role of these microRNAs in proliferative cardiovascular diseases. Under hypertrophy-inducing conditions SEQ ID NO: 11, 19 to 24, hsa-miR-493-5p, and hsa-miR-509 inhibit cardiomyocyte cell growth, which indicates an antihypertrophic phenotype and a role of these microRNAs in cardiovascular diseases related to apoptosis and anti-proliferation, i.e. proliferation inhibiting.

For microRNA from the group consisting of SEQ ID NO: 17, 19 to 22 no expression in the cardiovascular system is published. SEQ ID NO: 17 is expressed in the kidney. SEQ ID NO: 19 to 22 are expressed in placenta, spleen, kidney and testis.

It is surprising that SEQ ID NO: 17, 19 to 22 nevertheless affect cardiovascular tissue and modify cell size of cardiomyocytes.

In a preferred embodiment, the cardiovascular disease is related to induction and/or inhibition of cardiomyocyte cell growth. In another preferred embodiment, the cardiovascular disease or the predisposition thereof is a disease selected from the group consisting of cardiac hypertrophy, hypertensive heart failure, diastolic heart failure, systolic heart failure, heart-related storage disease, cardiomyopathy, such as M. Fabry, cardiomyopathies, e.g. dilatative cardiomyopathy, hypertrophic cardiomyopathy with and without obstruction, restrictive cardiomyopathy, arrhythmogenic right ventricular cardiomyopathy and other forms of cardiomyopathy, like diabetic cardiomyopathy, constrictive pericarditis, coronary artery disease, myocardial infarction, acute and chronic right heart failure, cardiac arrhythmias due to fibrosis, myocarditis-related fibrosis, diseases of the heart valves leading to valve stenosis or insufficiency (e.g. sclerosis), e.g. mitral valve stenosis and/or insufficiency, aortic valve stenosis and/or insufficiency, tricuspidal valve stenosis and/or insufficiency, pulmonary valve stenosis, blood vessel-related disease and/or insufficiency. The invention also concerns cardiac specific diseases involving fibrosis.

In a certain embodiment the manufactured medicament provided herein comprises one or more additional pharmaceutical agents, like diuretics (e.g. sprionolactone, eplerenone, furosemide), inotropes (e.g. dobutamine, milrinone), digoxin, vasodilators, angiotensin II converting enzyme (ACE) inhibitors (e.g. captopril, enalapril, lisinopril, benazepril, quinapril, fosinopril, and ramipril), angiotensin II receptor blockers (ARB) (e.g. candesartan, irbesartan, olmesartan, losartan, valsartan, telmisartan, eprosartan), calcium channel blockers, isosorbide dinitrate, hydralazine, nitrates (e.g. isosorbide mononitrate, isosorbide dinitrate), hydralazine, beta-blockers (e.g. carvedilol, metoprolol), and natriuretic peptides (e.g. nesiritide).

In a preferred embodiment the cardiovascular disease is a proliferative disease and the medicament comprises the microRNA selected from the group consisting of SEQ ID NO: 20, 21, 22, 24, and hsa-miR-509. In another preferred embodiment the cardiovascular disease is a proliferative disease and the medicament comprises the antisense against a microRNA selected from the group consisting of SEQ ID NO: 5 to 10, 13, 14, 17, and 18. In another preferred embodiment a proliferative disease is diagnosed and the microRNA is selected from the group consisting of SEQ ID NO: 5 to 10, 13, 14, 17, and 18.

In another preferred embodiment the cardiovascular disease relates to apoptosis or proliferation inhibition and the medicament comprises the microRNA selected from the group consisting of SEQ ID NO: 5 to 10, 13, 14, 17, and 18. In another preferred embodiment the cardiovascular disease relates to apoptosis or proliferation inhibition and the medicament comprises the antisense against a microRNA selected from the group consisting of SEQ ID NO: 20, 21, 22, 24, and hsa-miR-509. In another preferred embodiment a disease related to apoptosis or proliferation inhibition is diagnosed and the microRNA is selected from the group consisting of SEQ ID NO: 20, 21, 22, 24, and hsa-miR-509.

In a certain embodiment the proliferative disease includes heart weight increase, fibrosis related diseases, left ventricular dilation, cardiac hypertrophy, hypertensive heart failure, diastolic heart failure, systolic heart failure, and impairment of fractional shortening. In a certain embodiment the cardiovascular disease related to apoptosis or proliferation inhibition includes hypotrophy, insufficiency, heart failure, pericarditis, and infarction.

The term "proliferative disease" as used herein refers to diseases related to proliferation of tissue and organs. Preferably cell size is increased in a proliferative disease. In addition, proliferation includes increased number of cells and rearrangement of cells. The term "proliferative disease" also encompasses acute and chronic proliferation, localized disseminated and systemic proliferation, fibrosis, cell immigration, increased cell division, and preferably cellular growth and increase.

The term "apoptosis" as used herein refers to cell death that involves a series of biochemical events leading to a variety of morphological changes, like blebbing, cell membrane modification, nuclear fragmentation, chromatin condensation, chromosomal DNA fragmentation, and cell shrinkage. Also included are processes of disposal of cellular debris. The term "apoptosis" as used herein comprises not only physiological cell death and lysis, but also necrosis, defective and pathological apoptosis, and hypothropy, such as in ischemic damage.
The term "proliferation inhibition" as used herein refers to reduction of cell proliferation. Proliferation inhibition occurs by e.g. a decreased growth rate, shrinkage, and/or reduced cell division.

Under basal conditions SEQ ID NO: 5 to 10, 13, 14, 17, and 18 induced strong promotion of cardiomyocyte cell growth (about 1,6 to 2,2 fold), which indicates a particular prohypertrophic phenotype and an important role of these microRNAs in proliferative cardiovascular diseases. Under hypertrophic conditions SEQ ID NO: 20, 21, 22, 24, and hsa-miR-509 induced strong inhibition of cardiomyocyte cell growth (about 0,7 to 0,4 fold), which indicates a particular antihypertrophic phenotype and an important role of these microRNAs in cardiovascular diseases related to apoptosis and proliferation inhibition.

In another aspect the present invention relates to a method for diagnosing a cardiovascular disease, the method comprises the steps of:
(a) providing a sample of a patient supposed to suffer from the cardiovascular disease; and
(b) measuring a level of an endogenous microRNA of the sample,
wherein an modified level of a microRNA selected from the group consisting of SEQ ID NO: 1, 5 to 18, hsa-miR-18a*, hsa-miR-191*, hsa-miR-493-5p, hsa-miR-548a, and hsa-miR-767-5p, in comparison to a control sample indicates a proliferative cardiovascular disease or a predisposition thereof, and/or
an modified level of a microRNA selected from the group consisting of SEQ ID NO: 5, 11, 19 to 24, hsa-miR-493-5p, and hsa-miR-509, in comparison to a control sample indicates a cardiovascular disease that relates to apoptosis or proliferation inhibition or a predisposition thereof.

The term "providing a sample" refers to any kind of sample taking and sample preparation. The term "measuring a level" as used herein includes determining a concentration, an expression level, and a relative or absolute amount of endogenous microRNA of the sample. The term "modification" as used herein includes alteration, decrease, and increase.

In another aspect the present invention relates to a method for screening a pharmaceutically active compound for the treatment and/or prevention of a cardiovascular disease or a predisposition thereof, the method comprises the steps of:
(a) providing a sample containing a microRNA selected from the group consisting of SEQ ID NO: 1, 5 to 24, hsa-miR-18a*, hsa-miR-191*, hsa-miR-493-5p, hsa-miR-548a, hsa-miR-767-5p, and hsa-miR-509;
(b) contacting a candidate substance with the sample; and
(c) determining the effect of the candidate substance on the sample;
wherein a modification of the microRNA indicates a pharmaceutically active compound.

### Methods

### 1. Isolation of primary cells

Neonatal rat cardiomyocytes (NRCM) were isolated from 1-2 days old sprague dawley rats via enzymatic digestion. All preparation steps were performed under sterile conditions. The hearts were taken out and transferred into 10 cm cell culture dishes containing CBFHH and put on ice. After removing the atria, the hearts were cut into small pieces and treated with trypsin solution at room temperature (RT) for 15 min while stirring. Afterwards, the trypsin solution was exchanged and the tissue stirred again for 10 min. The suspension was carefully pipetted up and down 10 times, and the supernatant was transferred into a new tube containing 7.5 ml fetal bovine serum (FBS). This procedure was repeated until the trypsin solution was emptied. The tubes were subsequently centrifuged (800 x g at RT for 10 min). Each pellet containing the cells was resuspended in 10 ml of NRCM preplating medium, and the whole suspension was filtrated into a new 50 ml tube using a cell strainer (40 µm). For preplating, 10 ml of the filtrate were transferred in each of four 10 cm cell culture plates and incubated in a humidified 37 °C / 1 % CO₂ incubator for 1 h. During this incubation step, the more rapidly adherent fibroblasts were separated from the cardiomyocytes. After preplating, the cell supernatant containing the cardiomyocytes was transferred into a new 50 ml tube and the cell number was counted. Therefore, 50 µl of cell suspension were mixed with 50 µl trypan blue and the cell number was determined by counting 5 big squares of a "Fuchs-Rosenthal" cell counting chamber and multiplying the resulting cell number by two. Finally, 40.000 cells per well were sown on black 96-well plates (lbidi, Martinsried, Germany) containing NRCM medium with 1% FBS and cultured in a humidified 37 °C/1% CO₂ incubator.

### 2. Hypertrophy assay

NRCM were isolated and plated as described above (see 1. Isolation of primary cells). 24 h after isolation, the cells were transfected with the pre-miR^{™} miRNA Precursor Library (final concentration 50 nM; Ambion, Austin, USA) containing 471 human precursor miRNAs mimicking precursors annotated in miRBase sequence database version 8.0 (http://microrna.sanger.ac.uk/sequences). Before transfection the medium was replaced by NRCM medium with 5% FBS without antibiotics. The transfection procedure using Lipofectamine^{™} 2000 (Invitrogen, Karlsruhe, Germany) was performed according to manufacturers' instructions. After 4-6 h incubation in a humidified 37 °C/1% CO₂ incubator medium was exchanged to NRCM medium with 0,1% FBS. To induce hypertrophy one part of the cells was stimulated with 50 µM phenylephrine (PE) diluted in NRCM medium with 0.1% FBS after 48 h. For the unstimulated control cells, the medium was exchanged to NRCM medium with 0.1% FBS. Cells were incubated for 48 h and afterwards fixed by addition of 50 µl 4% paraformaldehyde (PFA) per well and 5 min incubation at 4 °C. Cells were washed three times with 1x PBS at RT and kept at 4 °C until staining. To stain the cells, they were incubated with 50 µl 0.2% Triton-X (diluted in 1 x PBS) per well for 5 min at RT and afterwards washed three times with 1x PBS. They were then incubated with 50 µl anti-α-actinin antibody (Sigma-Aldrich, Taufkirchen, Germany) solution (1:1000 diluted in 1x PBS) per well at 37°C for 30 min and washed three times with 1x PBS. They were then incubated with 50 µl of a mixture of alexa 488-conjugated goat anti-mouse IgG (final concentration 20 µg/ml; Invitrogen, Karlsruhe, Germany), DRAQ5^{™} red-fluorescent DNA probe (10 µM; Biostatus Limited, Leicestershire, UK) and DAPI (100 µg/µl; Sigma-Aldrich, Taufkirchen, Germany), all diluted in 1x PBS, at 37°C for 30 min. After three washing steps with 1x PBS, cells were covered with 100 µl 50% glycerol solution per well and stored at 4 °C. Cell sizes were determined by using automated fluorescence microscopy (X-Cite^{R}120 illumination system, EXFO, Munich, Germany; SPOT PURSUIT CCD camera, Visitron Systems, Puchheim, Germany; BD Carv II, BD Biosciences, Heidelberg, Germany; AxioObserver.Z1, Zeiss, Göttingen, Germany) and computerized analysis (MetaMorph Basic imaging software, Molecular Devices, Ismaning, Germany).

**Table 1. Candidate miRNAs that induce cardiomyocyte cell growth.**

| **miRNA (Sanger) /** | **SEQ ID NO:** | **Cell size normalized to control** | | **Tissue expression** | |
|---|---|---|---|---|---|
| | | **Basal** | **50** µ**M PE** | **Cardial** | **Other organs** |
| hsa-miR-130a | | 1.19 | 0.96 | | brain (cerebellum)¹; lung ² |
| hsa-miR-130b | | 1.17 | 0.87 | heart ³ | liver, colon ³ |
| hsa-miR-132 | | 1.32 | 1.02 | | brain (cerebellum¹, frontal cortex ³) |
| hsa-miR-137 ^{B} | 25 | 1.43 | 0.97 | | brain (cortex ^{1,3}); lung, bladder, ovary ³ |
| hsa-miR-140 | | 1.18 | 0.91 | | small intestine ¹; uterus, fallopian, ovary ³ |
| hsa-miR-181b | | 1.16 | 0.91 | | brain (frontal cortex) ³ |
| hsa-miR-181c | | 1.19 | 0.86 | | colon, brain ³ |
| **hsa-miR-181d** | 1 | 1.17 | 0.90 | heart ⁵ | brain, thymus ⁵ |
| hsa-miR-184 | | 1.26 | 1.00 | heart³ | liver, skeletal muscle ³; placenta ⁵ |
| hsa-miR-189 | | 1.15 | 0.92 | | ovary ³ |
| hsa-miR-18a | 2 | 1.19 | 0.87 | | spleen ¹ |
| **hsa-miR-18a*** | | 1.35 | 1.06 | | |
| hsa-miR-190 | | 1.21 | 0.89 | | kidney, brain ³ |
| hsa-miR-191 | 3 | | | | |
| **hsa-miR-191*** | | 1.41 | 1.00 | | |
| hsa-miR-192 | | 1.16 | 0.94 | | liver ^{2, 3}; small intestine ³; epithelium ⁵ |
| hsa-miR-19a | | 1.29 | 1.04 | | colon, bone marrow, ovary ³ |
| hsa-miR-19b | | 1.24 | 1.04 | | spleen ^{1, 2, 3}; colon, bone marrow, ovary ³ |
| hsa-miR-210 | | 1.33 | 1.08 | heart ³ | |
| hsa-miR-26a | | 1.17 | 0.87 | | brain (cerebellum, midbrain ¹) ²; skeletal muscle, ovary, uterus ³ |
| hsa-miR-26b | | 1.30 | 0.93 | | liver, brain (cortex, cerebellum)¹; lung ²; ovary, uterus ³ |
| hsa-miR-28 | | 1.24 | 0.99 | | ovary, uterus ³ |
| hsa-miR-29a | | 1.98 | 1.59 | heart³ | colon, brain (cerebellum ¹); brain ²; spleen, ovary, uterus ³ |
| hsa-miR-29b | | 1.44 | 1.11 | heart³ | colon, brain (cortex, midbrain ¹)³; brain ²; spleen, ovary ³ |
| hsa-miR-29c | | 1.40 | 1.08 | heart³ | brain (cortex, midbrain ¹); brain ², ovary ³ |
| hsa-miR-301 | | 1.29 | 0.95 | heart³ | bone marrow, liver ³ |
| hsa-miR-302b* | | 1.16 | 0.96 | low abun-dance ⁵ | |
| hsa-miR-30a-5p | | 1.16 | 1.04 | | spleen, brain (cortex, midbrain) ¹ |
| hsa-miR-31 | | 1.20 | 0.95 | | colon ³; epithelium ⁵ |
| hsa-miR-32 | | 1.55 | 1.23 | | cervix, thymus, skeletal muscle, lung, ovary, uterus ³ |
| hsa-miR-324 | 26 | | | | |
| hsa-miR-324-5p ^{B} | | 1.20 | 0.97 | heart³ | cervix, brain, bladder, ovary ³ |
| hsa-miR-326 | | 1.44 | 1.28 | heart³ | brain, skeletal muscle ³ |
| hsa-miR-330 | | 1.68 | 1.20 | | brain (frontal cortex) ^{3,5} |
| hsa-miR-382 ^{B} | 27 | 1.23 | 1.04 | | brain, placenta ⁴ |
| hsa-miR-493 | 4 | | | | |
| **hsa-miR-493-5p ^{A}** | | 1.23 | 0.73 | | |
| hsa-miR-500 | | 1.63 | 1.04 | | kidney, placenta ⁵ |
| hsa-miR-505 | | 1.71 | 1.12 | | liver ⁵ |
| hsa-miR-507 | | 1.57 | 0.94 | | testis ⁵ |
| hsa-miR-527 | | 1.76 | 1.15 | | placenta ⁵ |
| **hsa-miR-544** | 5 | 1.64 | 1.08 | | |
| **hsa-miR-548 a-1, a-2, a-3** | 6, 7, 8 | | | | |
| **hsa-miR-548a** | | 1.73 | 1.04 | | |
| **hsa-miR-552** | 9 | 1.81 | 1.03 | | |
| **hsa-miR-555 ^{B}** | 10 | 1.89 | 1.14 | | |
| **hsa-miR-578 ^{A}** | 11 | 1.20 | 0.64 | | |
| **hsa-miR-600** | 12 | 1.21 | 0.91 | | |
| **hsa-miR-608** | 13 | 1.73 | 1.09 | | |
| **hsa-miR-609** | 14 | 1.78 | 0.97 | | |
| **hsa-miR-617 ^{B}** | 15 | 1.24 | 1.00 | | |
| **hsa-miR-634** | 16 | 1.31 | 0.97 | | |
| **hsa-miR-660** | 17 | 1.68 | 1.12 | | kidney ⁶ |
| **hsa-miR-767** | 18 | | | | |
| **hsa-miR-767-5p** | | 2.15 | 1.25 | | |

**Table 2. Candidate miRNAs that inhibit cardiomyocyte cell growth.**

| **miRNA** | **SEQ ID NO:** | **Cell size normalized to control** | | **Tissue expression** | |
|---|---|---|---|---|---|
| | | **Basal** | **50** µ**M PE** | **Cardial** | **Other organs** |
| hsa-miR-134 | | 0.82 | 0.82 | | brain (cortex) ^{1,3}; ovary ³ |
| hsa-miR-144 | | 0.77 | 0.72 | heart¹ | colon¹; spleen ²; bone marrow ³ |
| hsa-miR-148a | | 0.96 | 0.72 | | spleen ¹, liver ² |
| hsa-miR-148b | | 0.95 | 0.73 | | spleen, frontal cortex, lung ³ |
| hsa-miR-152 | | 1.00 | 0.80 | | colon ^{1,3}, ovary ³ |
| hsa-miR-182* | | 0.73 | 0.65 | heart³ | liver, skeletal muscle ³ |
| hsa-miR-183 | | 0.66 | 0.77 | heart³ | thymus, liver ³ |
| **hsa-miR-18b** | 19 | 1.07 | 0.79 | | placenta, spleen, kidney ⁶ |
| hsa-miR-365 | | 0.95 | 0.54 | heart ⁵ | adipose tissue ⁵ |
| hsa-miR-422a | | 0.83 | 0.79 | heart ⁵ | skeletal muscle ⁵ |
| hsa-miR-422b | | 0.79 | 0.76 | heart ^{2,4} | skeletal muscle ⁴ |
| **hsa-miR-493-5p ^{A}** | | 1.23 | 0.73 | | |
| hsa-miR-506 | | 0.38 | 0.30 | | testis ⁵ |
| **hsa-miR-509 -1, -2, -3** | 20, 21, 22 | | | | |
| **hsa-miR-509 ^{B}** | | 0.79 | 0.58 | | testis ⁵ |
| **hsa-miR-571** | 23 | 1.11 | 0.72 | | |
| **hsa-miR-578 ^{A}** | | 1.20 | 0.64 | | |
| **hsa-miR-605** | 24 | 0.86 | 0.45 | | |

| | | | | | |
|---|---|---|---|---|---|
| ^{A} These miRNAs have an opposite effect under basal and hypertrophy-inducing (50 µM PE) condition. ^{B} These miRNAs display a specific morphological phenotype. ¹ M. Lagos-Quintana et al., 2002 ² K. Wang et al., 2009 ³ S. Baskerville and DP. Bartel, 2005 ⁴ EJ. Lee et al., 2007 ⁵ Y. Liang et al., 2007 ⁶ http://mirnamap.mbc.nctu.edu.tw/ empty cells: no data ^{*} asterisk: antisense oligonucleotide hsa-miR-299: SEQ ID NO: 28 | | | | | |

The mature miRNA hsa-mir-xyz-5p emerges from the precursor molecule of hsa-mir-xyz. Three genes (hsa-mir-548a-1, hsa-mir-548a-2 and hsa-mir-548a-3) are coding for hsa-mir-548a. The mature hsa-mir-548a molecule emerges from the precursor molecules of all three genes. Three genes (hsa-mir-509-1, hsa-mir-509-2 and hsa-mir-509-3) are coding for hsa-mir-509. The mature hsa-mir-509 molecule emerges from the precursor molecules of all three genes.

### References

Ambros, V. The functions of animal microRNAs. Nature 431, 350-355 (2004).
Bartel, D. P. MicroRNAs: Genomics, biogenesis, mechanism, and function. Cell 116, 281-297 (2004, A).
Bartel, D. P. et al. Micromanagers of gene expression: The potentially widespread influence of metazoan microRNAs. Nature Rev. Genet. 5, 396-400 (2004, B).
Baskerville, S., Bartel DP. Microarray profiling of microRNAs reveals frequent co-expression with neighboring miRNAs and host genes. RNA 11, 241-7 (2005).
Care, A. et al. MicroRNA-133 controls cardiac hypertrophy. Nat Med 13, 613-618 (2007).
Kim, V.N. MicroRNA biogenesis: Coordinated cropping and dicing, Nature Reviews 6, 376-385 (2005).
Kruetzfeldt, J. et al. Silencing of microRNAs in vivo with 'antagomirs'. Nature 438, 685-689 (2005).
Lagos-Quintana, M. et al. Identification of Tissue-Specific MicroRNAs from Mouse. Current Biology 12, 735-739 (2002).
Lee, E.J. et al. Systematic evaluation of microRNA processing patterns in tissues, cell lines, and tumors. RNA 14, 35-42 (2007).
Lewis, B. P., et al. Prediction of mammalian microRNA targets. Cell 115, 787-798 (2003).
Lewis, B. P., et al. Conserved seed pairing, often flanked by adenosines, indicates that thousands of human genes are microRNA targets. Cell 120, 15-20 (2005).
Liang, Y. et al. Characterization of microRNA expression profiles in normal human tissues. BMC Genomics 8, 166-186 (2007).
Mi, S. et al. MicroRNA expression signatures accurately discriminate acute lymphoblastic leukemia from acute myeloid leukemia. Proc. Natl. Acad. Sci. U S A 104, 19971-19976 (2007).
Wang, K. et al. Circulating microRNAs, potential biomarkers for drug-induced liver injury. PNAS, doi:10.1073/pnas.0813371106 (published online before print February 25, 2009).
Yang, B. et al. The muscle-specific microRNA miR-1 regulates cardiac arrhythmogenic potential by targeting GJA1 and KCNJ2. Nat Med 13, 486-491 (2007). http://mirnamap.mbc.nctu.edu.tw/

## Claims

1. Use of a microRNA selected from the group consisting of SEQ ID NO: 10, 15, 20, 21, 22, 25, 27, 28, hsa-miR-299-5p, and hsa-miR-509 for the manufacture of a medicament for the treatment and/or prevention of a disease selected from the group consisting of lesions, injury, neuronal degeneration, paraplegia, cardiovascular disease, and cicatrization of an organ, connective tissue or skin.

2. Use of a microRNA selected from the group consisting of SEQ ID NO: 10, 15, 20, 21, 22, 25 to 28, hsa-miR-299-5p, hsa-miR-324-5p, and hsa-miR-509 for the manufacture of an implant for plastic surgery, organ and/or tissue replacement.

3. An implant obtained by a method comprising the steps of:
(a) providing at least one cell; and
(b) contacting a microRNA selected from the group consisting of SEQ ID NO: 10, 15, 20, 21, 22, 25 to 28, hsa-miR-299-5p, hsa-miR-324-5p, and hsa-miR-509 with the cell;
wherein a modification of a morphological phenotype of the cell is induced.

4. Use of a microRNA or an antisense oligonucleotide against the microRNA for inducing a modification of a morphological phenotype of a cell, wherein the microRNA is selected from the group consisting of SEQ ID NO: 10, 15, 20, 21, 22, 25 to 28, hsa-miR-299-5p, hsa-miR-324-5p, and hsa-miR-509.

5. Use of claim 4, wherein the modification is selected from the group consisting of reshaping, elongation, branching, rounding, protuberance and cytoskeletal reorganization.

6. Use of claim 4 or 5, wherein the microRNA or the antisense against the microRNA is used to enhance or inhibit elongation and branching, and the microRNA is selected from the group consisting of SEQ ID NO: 10, 15, 20, 21, 22, 26, 27, 28, hsa-miR-299-5p, hsa-miR-324-5p, and hsa-miR-509.

7. Use of claim 4 or 5, wherein SEQ ID NO: 25 or the antisense against SEQ ID NO: 25 is used to reorganize the cytoskeleton.

8. Use of a microRNA selected from the group consisting of SEQ ID NO: 1, 5 to 24, hsa-miR-18a*, hsa-miR-191*, hsa-miR-493-5p, hsa-miR-548a, hsa-miR-767-5p, hsa-miR-509, an antisense oligonucleotide against SEQ ID NO: 1, 5 to 24, hsa-miR-493-5p, hsa-miR-548a, hsa-miR-767-5p, and an antisense oligonucleotide against hsa-miR-509 for the manufacture of a medicament for the treatment and/or prevention of a cardiovascular disease.

9. Use of a microRNA selected from the group consisting of SEQ ID NO: 1, 5 to 24, hsa-miR-18a*, hsa-miR-191*, hsa-miR-493-5p, hsa-miR-548a, hsa-miR-767-5p, hsa-miR-509, an antisense oligonucleotide against SEQ ID NO: 1, 5 to 24, hsa-miR-493-5p, hsa-miR-548a, hsa-miR-767-5p, and an antisense oligonucleotide against hsa-miR-509 for the diagnosis of a cardiovascular disease or a predisposition thereof.

10. Use of claim 8 or 9, wherein the cardiovascular disease or the predisposition thereof is a disease selected from the group consisting of cardiac hypertrophy, hypertensive heart failure, diastolic heart failure, systolic heart failure, heart-related storage disease, cardiomyopathy, constrictive pericarditis, coronary artery disease, acute myocardial infarction, chronic myocardial infarction, right heart failure, cardiac arrythymias, myocarditis-related fibrosis, heart valve disease, and blood vessel-related disease.

11. Use of claim 10, wherein the cardiovascular disease is a proliferative disease and the medicament comprises the microRNA selected from the group consisting of SEQ ID NO: 19, 20, 21, 22, 24, and hsa-miR-509.

12. Use of claim 10, wherein the cardiovascular disease relates to apoptosis or proliferation inhibition and the medicament comprises the microRNA selected from the group consisting of SEQ ID NO: 5 to 10, 13, 14, 17, and 18.

13. A method for diagnosing a cardiovascular disease, the method comprises the steps of:
(a) providing a sample of a patient supposed to suffer from the cardiovascular disease; and
(b) measuring a level of an endogenous microRNA of the sample,
wherein a modified level of a microRNA selected from the group consisting of SEQ ID NO: 1, 5 to 18, hsa-miR-18a*, hsa-miR-191*, hsa-miR-493-5p, hsa-miR-548a, and hsa-miR-767-5p, in comparison to a control sample indicates a proliferative cardiovascular disease or a predisposition thereof, and/or a modified level of a microRNA selected from the group consisting of SEQ ID NO: 5, 11, 19 to 24, hsa-miR-493-5p, and hsa-miR-509, in comparison to a control sample indicates a cardiovascular disease that relates to apoptosis or proliferation inhibition or a predisposition thereof.

14. A method for screening a pharmaceutically active compound for the treatment and/or prevention of a cardiovascular disease or a predisposition thereof, the method comprises the steps of:
(a) providing a sample comprising a microRNA selected from the group consisting of SEQ ID NO: 1, 5 to 24, hsa-miR-18a*, hsa-miR-191*, hsa-miR-493-5p, hsa-miR-548a, hsa-miR-767-5p, and hsa-miR-509;
(b) contacting a candidate substance with the sample; and
(c) determining the effect of the candidate substance on the sample;
wherein a modification of the microRNA indicates a pharmaceutically active compound.
